(19) Europäisches Patentamt
European Patent Office
Office européen des brevets

(11) **EP 4 316 649 A1**

(12) **EUROPEAN PATENT APPLICATION**
published in accordance with Art. 153(4) EPC

(43) Date of publication:
**07.02.2024 Bulletin 2024/06**

(21) Application number: **22780377.2**

(22) Date of filing: **23.03.2022**

(51) International Patent Classification (IPC):
**B01J 23/44** (2006.01)     **B01J 35/10** (2006.01)
**C07B 61/00** (2006.01)     **C07C 5/05** (2006.01)
**C07C 13/12** (2006.01)

(52) Cooperative Patent Classification (CPC):
**B01J 23/44; B01J 35/60; C07B 61/00; C07C 5/05;
C07C 13/12**

(86) International application number:
**PCT/JP2022/013503**

(87) International publication number:
**WO 2022/210162 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2021   JP 2021062072**

(71) Applicant: **Zeon Corporation
Tokyo 100-8246 (JP)**

(72) Inventors:
• **NISHINOHARA, Chihiro**
  **Yonezawa-shi, Yamagata 992-1128 (JP)**
• **SASANUMA, Takashi**
  **Tokyo 100-8246 (JP)**

(74) Representative: **Maiwald GmbH
Elisenhof
Elisenstraße 3
80335 München (DE)**

(54) **CATALYST, AND METHOD FOR PRODUCING CYCLOPENTENE**

(57)    The purpose of the present invention is to provide a catalyst that enables a hydrogenation reaction of cyclopentadiene to cyclopentene in a gas phase that exhibits both a high conversion rate and high selectivity, and a method for producing the cyclopentene in a gas phase that exhibits both a high conversion rate and high selectivity. A catalyst according to the disclosure and a method for producing cyclopentadienecyclopentene according to the disclosure are a catalyst and a method for producing cyclopentene using the catalyst that is used in a hydrogenation reaction of cyclopentadiene in a gas phase to form the cyclopentene, the catalyst including palladium (Pd) and a titanium dioxide ($TiO_2$) support, wherein the titanium dioxide ($TiO_2$) support contains anatase-type titanium dioxide ($TiO_2$).

*FIG. 1*

EP 4 316 649 A1

**Description**

TECHNICAL FIELD

This disclosure relates to a catalyst, and a method for producing cyclopentene.

BACKGROUND

Cyclopentene (hereinafter abbreviated as "CPE") is a useful compound in the chemical industry, and is used as a raw material compound for conversion into value-added compounds through various reactions such as oxidation reactions, hydration reactions, and Diels-Alder reactions, for example. The CPE can be produced by separation and generation of a petroleum fraction, and at the same time, cyclopentadiene (hereinafter abbreviated as "CPD") can also be obtained as a fractional mixture. The CPD can degrade a catalyst, or the like, and thereby reduce the efficiency of the various reactions using the CPE as the raw material compound. Therefore, in order to improve the efficiency of the various reactions using the CPE as the raw material compound, the CPD is reduced (hydrogenated) and converted into the CPE.

As methods for reducing the CPD to obtain the CPE, (a) a batch hydrogenation method in which, after the CPD, an ammonia aqueous solution, and Rane-Nickel are fed into a reactor in advance, hydrogen is supplied while stirring (Patent Literature (PTL) 1), (b) a batch reaction method in which, after the CPD, deionized water, Raney nickel, and monoethanol amine are fed into a reactor in advance and heated, hydrogen is supplied (Patent Literature (PTL) 2), (c) a method in which, after deionized water, Raney nickel, and monoethanol amine are fed into a reactor, the CPD and hydrogen are fed while controlling the concentration of the CPD in reaction solution to be a predetermined level or less (Patent Literature (PTL) 3) are known.

However, the method (a) above has the disadvantage of using ammonia to obtain the cyclopentene in a high yield rate, which causes operational difficulties or complicates facilities. In the method (b) above, increasing the amount of the catalyst to shorten reaction time results in a lower yield rate. In the method (c) above, the CPE can be obtained in a high yield rate and high selectivity, but a reaction rate is not fast enough and productivity is somewhat low.

The above methods (a), (b), and (c) are performed in a liquid phase, but facilities are complicated in a liquid phase system. On the other hand, it is advantageous to conduct reactions in a gas phase because it is expected to reduce the number of devices and to enable gas-phase continuous hydrogenation reactions that can exhibit high productivity without complicating facilities. As a method in the gas phase, (d) a continuous hydrogenation method in which hydrogen and the CPD are circulated in a reaction tube with a palladium-containing catalyst therein, to perform a gas-phase hydrogenation reaction is known. However, in the method (d) above, there is a problem in controlling the activity of Pd, and cyclopentane, which is a perhydrogenated product of the CPD, is formed as a byproduct. In addition, the cyclopentane and the CPE have close boiling points and are extremely difficult to separate, so it is necessary to increase selectivity in the industrial production. Therefore, it is not possible to realize both a high conversion rate from the CPD to the CPE and high selectivity to selectively produce the CPE while suppressing the byproduction of the perhydrogenated product (cyclopentane), resulting in poor productivity.

CITATION LIST

Patent Literature

PTL 1: JPS5476549U
PTL 2: JP2001261592A
PTL 3: JP2004175685A

SUMMARY

(Technical Problem)

As described above, performing a method of reducing CPD to obtain CPE in a gas phase, instead of in a liquid phase, is advantageous from the viewpoint of improving productivity with fewer number of devices. Therefore, there is a need for a method for conducting a hydrogenation reaction of the CPD in the gas phase that exhibits both a high conversion rate from the CPD to the CPE and high selectivity for selectively producing the CPE, while suppressing the byproduction of a perhydrogenated product (cyclopentane), which is suitable for the industrial production of the CPE, and a suitable catalyst for the method.

It would be helpful to provide a catalyst that enables a hydrogenation reaction of CPD to CPE in a gas phase that exhibits both a high conversion rate and high selectivity, and a method for producing the CPE in a gas phase that exhibits both a high conversion rate and high selectivity.

(Solution to Problem)

The inventors have made a diligent study with the aim of solving the above problem. The inventors have found that by using a supported composite in which palladium (Pd) is supported on an anatase-containing titanium dioxide (TiOz) support as a catalyst for a hydrogenation reaction of CPD to CPE in a gas phase, the CPE is produced with both a high conversion rate and high selectivity, and have completed the disclosure.

In other words, this disclosure aims to advantageously solve the above problem. A catalyst according to the disclosure is a catalyst used in a hydrogenation reaction of cyclopentadiene in a gas phase to form cyclopentene, the catalyst including palladium (Pd) and a titanium dioxide (TiOz) support, wherein the titanium dioxide (TiOz) support contains anatase-type titanium dioxide (TiOz). This catalyst enables both a high conversion rate and high selectivity in the hydrogenation reaction of the CPD to the CPE in the gas phase.

In the catalyst according to the disclosure, it is preferable that

(i) the content of titanium dioxide (TiOz) relative to the total weight of the catalyst is 85 weight% or more and 99.9 weight% or less, and
(ii) the content of the palladium (Pd) relative to the weight of the titanium dioxide (TiOz) support is 0.1 weight% or more and 2 weight% or less. In the catalyst according to the disclosure, it is more preferable that
(i') the content of the titanium dioxide (TiOz) relative to the total weight of the catalyst is 95 weight% or more and 99.9 weight% or less, and
(ii') the content of the palladium (Pd) relative to the weight of the titanium dioxide (TiOz) support is 0.5 weight% or more and 1 weight% or less. Higher conversion rates and higher selectivity in the hydrogenation reactions can be obtained when the contents of the titanium dioxide (TiOz) and the palladium (Pd) in the catalyst are in the above ranges.

In the catalyst according to the disclosure, it is preferable that the titanium dioxide ($TiO_2$) has an anatase ratio of 80% or more. In addition, in the catalyst according to the disclosure, it is more preferable that the titanium dioxide ($TiO_2$) has an anatase ratio of 95% or more. The higher the anatase ratio of the titanium dioxide ($TiO_2$), the higher the conversion rate and the higher the selectivity in the hydrogenation reaction.

It is preferable that the catalyst according to the disclosure further contains sulfur trioxide ($SO_3$). In this case, it is more preferable that the content of the sulfur trioxide ($SO_3$) relative to the total weight of the titanium dioxide ($TiO_2$) and the sulfur trioxide ($SO_3$) in the catalyst is 0.1 weight% or more and 5.0 weight% or less. Since the catalyst according to the disclosure contains the sulfur trioxide ($SO_3$), higher selectivity can be obtained in the hydrogenation reaction.

A method for producing cyclopentene according to the disclosure includes a step of conducting a hydrogenation reaction of cyclopentadiene in a gas phase to form the cyclopentene in the presence of the catalyst according to the disclosure. The use of the catalyst according to the disclosure enables both a high conversion rate and high selectivity in the hydrogenation reaction, and improves the production efficiency of the CPE.

(Advantageous Effect)

The present disclosure enables a hydrogenation reaction of CPD to CPE in a gas phase that exhibits both a high conversion rate and high selectivity.

BRIEF DESCRIPTION OF THE DRAWINGS

In the accompanying drawings:

FIG. 1 illustrates an example of a reaction device for producing CPE based on a method according to the disclosure;
FIG. 2 is a diagram illustrating the performance of hydrogenation catalysts with different anatase ratios;
FIG. 3A is an (overlaid) diagram illustrating the performance of hydrogenation catalysts with palladium (Pd) on different supports;
FIG. 3B is a diagram illustrating the performance of hydrogenation catalysts with palladium (Pd) on titanium dioxide (TiOz) supports;
FIG. 3C is diagram illustrating the performance of hydrogenation catalysts with palladium (Pd) on activated carbon supports;
FIG. 3D is diagram illustrating the performance of hydrogenation catalysts with palladium (Pd) on aluminum oxide ($Al_2O_3$) supports;
FIG. 3E is diagram illustrating the performance of hydrogenation catalysts with palladium (Pd) on zinc oxide (ZnO) supports; and
FIG. 3F is diagram illustrating the performance of hydrogenation catalysts with palladium (Pd) on silicon dioxide (SiOz) supports.

DETAILED DESCRIPTION

(CPD Hydrogenation Catalyst)

A catalyst of the disclosure is a catalyst used in a hydrogenation reaction of cyclopentadiene in a gas phase to produce cyclopentene. The catalyst contains palladium (Pd) and a titanium dioxide ($TiO_2$) support, and the titanium dioxide (TiOz) support contains anatase-type titanium dioxide (TiOz). The catalyst of the disclosure may optionally further contain other components. Such components include, for example, sulfur trioxide ($SO_3$). The catalyst of the disclosure is usually a catalyst (supported catalyst) having a structure in which palladium (Pd) is supported on a support containing titanium dioxide ($TiO_2$) as a main component.

<Support/Titanium Dioxide ($TiO_2$)>

The titanium dioxide (TiOz) is the main component of the support. The titanium dioxide (TiOz) contains an anatase type, as a crystal type. The anatase ratio of the titanium dioxide ($TiO_2$) is not particularly limited as long as the purpose of the disclosure is achieved, but may be normally 50% or more, preferably 80% or more, and more preferably 95% or more from the viewpoint of achieving both an excellent conversion rate and selectivity of the CPD hydrogenation reaction in the gas phase. The anatase ratio of the titanium dioxide ($TiO_2$) can be measured using XRD.
The basicity of the titanium dioxide (TiOz) is not particularly limited as long as the purpose of the disclosure is achieved, but may be, for example, 0.01 $\mu$mol/g or more, preferably 0.04 $\mu$mol/g or more, more preferably 0.1 $\mu$mol/g or more at 100 to 450°C. The basicity of the titanium dioxide (TiOz) can be measured using $CO_2$-TPD.
The acidity of the titanium dioxide (TiOz) is not particularly limited as long as purpose of the disclosure is achieved, but may be, for example, 1 $\mu$mol/g or less, preferably 0.5 $\mu$mol/g or less, more preferably 0.2 $\mu$mol/g or less at 100 to 450°C. The acidity of the titanium dioxide (TiOz) can be measured using $NH_3$-TPD.
The content of the titanium dioxide (TiOz) in the total weight of the catalyst is not particularly limited as long as the purpose of the disclosure is achieved, but may be preferably 85 weight% or more, more preferably 95 weight% or more, preferably 99.9 weight% or less, and more preferably 99.5 weight% or less.

<Palladium (Pd)>

When the catalyst of the disclosure is a supported catalyst, the palladium (Pd) is metal palladium (Pd) supported on a support. The metal palladium (Pd) supported on the support is preferably in the form of particles, and more preferably has an average particle diameter of 5 nm or more and 20 nm or less. The average particle diameter can be measured using the CO pulse adsorption method.
The metal specific surface area of the palladium (Pd) in the catalyst of the disclosure is not particularly limited as long as the purpose of the disclosure is achieved, but may be, for example, 0.05 $m^2$/g or more, preferably 0.5 $m^2$/g or more, and more preferably 0.1 $m^2$/g or more. When the metal specific surface area of the palladium (Pd) is equal to or above these lower limits, sufficient catalytic activity can be obtained.
In the disclosure, the "metal specific surface area" of the palladium (Pd) can be expressed as the surface area of palladium (Pd) metal in a state supported by a support ($m^2$ (palladium (Pd) metal)/g (palladium (Pd) metal)), and can be measured using the CO pulse adsorption method. The metal specific surface area of the palladium (Pd) can also be expressed as the ratio of the surface area of the palladium (Pd) metal relative to the weight of the titanium dioxide (TiOz), which serves as the support (the surface area ($m^2$) of the palladium (Pd) metal/the weight (g) of the titanium dioxide (TiOz)).
The content of the palladium (Pd) relative to the weight of the titanium dioxide (TiOz) support is not particularly limited as long as the purpose of the disclosure is achieved, but from the viewpoint of increasing activity and obtaining a sufficient hydrogenation rate, for example, 0.1 weight% or more, preferably 0.25 weight% or more, more preferably 0.45 weight% or more, and even more preferably, 0.5 weight% or more. The content of the palladium (Pd) relative to the total weight of the catalyst may be, for example, 2 weight weight% or less, preferably 1 weight weight% or less, more preferably 0.75 weight% or less, and even more preferably 0.55 weight% or less from the viewpoint of catalytic efficiency of Pd. The weight of the titanium dioxide ($TiO_2$) support refers to the total weight of the titanium dioxide ($TiO_2$) and any

trace components, impurities, or the like optionally contained in the titanium dioxide (TiOz) support.

<Other Components/Sulfur Trioxide (SO₃)>

The catalyst of the disclosure may optionally further contain other components. In particular, it is preferable that the catalyst of the disclosure further contains the sulfur trioxide (SO₃) as the other component. When the catalyst of the disclosure contains the sulfur trioxide (SO₃), the sulfur trioxide (SO₃) may be mixed into the catalyst of the disclosure as, for example, a trace component, an impurity, or the like contained in the titanium dioxide (TiOz) support. In such cases, what state of the sulfur trioxide (SO₃) is present in the catalyst is not known in detail, but it is assumed that the sulfur trioxide (SO₃) may be present in the form of, for example, a compound (e.g., titanyl sulfate) with the titanium dioxide (TiOz). The catalyst of the disclosure can obtain high selectivity by containing the sulfur trioxide (SO₃). The content ratio of the sulfur trioxide (SO₃) relative to the total weight of the titanium dioxide (TiO₂) and the sulfur trioxide (SO₃) in the catalyst is preferably 0.1 weight% or more, more preferably 0.5 weight% or more, even more preferably 1.0 weight% or more, preferably 5.0 weight% or less, more preferably 4.0 weight% or less, and even more preferably 3.0 weight% or less. The higher the content ratio of the sulfur trioxide (SO₃), the higher the selectivity. On the other hand, when the content ratio of the sulfur trioxide (SO₃) is too high, the function of the titanium dioxide (TiOz) support can be reduced, so the sulfur trioxide (SO₃) should be contained as a trace component equal to or below the above limits. The content of the sulfur trioxide (SO₃) can be measured, for example, by X-ray fluorescence analysis (XRF).

<Structure, Shape, and Component Compositions of Catalyst>

As described above, the catalyst of the disclosure usually has a structure in which the palladium (Pd) is supported on the support containing the titanium dioxide (TiOz) as the main component. Such a support structure includes, for example, the Eggshell type and the like. The form of the catalyst of the disclosure is not particularly limited as long as the purpose of the disclosure is achieved, but may be a pellet form, a sphere form, a cylinder form, a ring form, or the like. The pellet-form catalyst can be used as a fixed bed catalyst in a reaction vessel. The size (e.g., particle size) of the catalyst is not particularly limited, as an optimum value can be selected depending on an inner diameter or the like of a reaction tube. When the catalyst of the disclosure is in the pellet form, the pellet size may be, for example, 2 mm or more and 4 mm or less. When the catalyst of the disclosure is in the pellet form, it is preferable that the palladium (Pd) in the catalyst is in the form of particles, and it is more preferable that the particle size is 5 nm or more and 20 nm or less. When the catalyst of the disclosure is of the Eggshell type, the length of a palladium (Pd) present outer shell in the catalyst is preferably 10 $\mu$m or more and 150 $\mu$m or less.
In the disclosure, the "size (e.g., average particle diameter)" of the hydrogenation catalyst can be measured using the sieve test method according to JIS K0069 and represents a weight-average particle diameter.
The particle diameter of the palladium (Pd) in the catalyst can be measured using the CO-pulse adsorption method and represents a weight-average particle diameter.
The length of the palladium (Pd) present outer shell in the catalyst can be measured using SEM-EDX.
The specific surface area of the catalyst of the disclosure is not particularly limited as long as the purpose of the disclosure is achieved, but may be, for example, 20 $m^2$/g or more, preferably 40 $m^2$/g or more, more preferably 50 $m^2$/g or more.
In the disclosure, the "specific surface area" of the hydrogenation catalyst means a specific surface area measured using the BET adsorption method, and can be measured, for example, using a BET specific surface area meter.
The total pore volume of the catalyst of the disclosure is not particularly limited as long as the purpose of the disclosure is achieved, but may be, for example, 0.5 $cm^3$/g or less, preferably 0.3 $cm^3$/g or less, more preferably 0.25 $cm^3$/g or less from the viewpoint of suppressing side reactions.
In the disclosure, the "total pore volume" of the hydrogenation catalyst can be measured, for example, using the BET adsorption method.
The average pore diameter of the catalyst of the disclosure is not particularly limited as long as the purpose of the disclosure is achieved, but may be, for example, 100 nm or less, preferably 50 nm or less, more preferably 30 nm or less.
In the disclosure, the "average pore diameter" of the hydrogenation catalyst can be measured, for example, using the BET adsorption method.
The palladium (Pd) metal dispersibility in the catalyst of the disclosure is not particularly limited as long as the purpose of the disclosure is achieved, but may be, for example, 0.1% or more, preferably 1% or more, and more preferably 10% or more.
In the disclosure, the "metal dispersibility" of the hydrogenation catalyst can be measured, for example, using the CO-pulse adsorption method.
As component compositions in the catalyst of the disclosure, it is preferable that (i) the content of the titanium

dioxide (TiO$_2$) relative to the total weight of the catalyst is 85 weight% or more and 99.9 weight% or less, and (ii) the content of the palladium (Pd) relative to the total weight of the catalyst is 0.1 weight% or more and 2 weight% or less. As the component compositions of the catalyst of the disclosure, it is more preferable that (i') the content of the titanium dioxide (TiOz) relative to the total weight of the catalyst is 95 weight% or more and 99.9 weight% or less, and (ii') the content of the palladium (Pd) relative to the total weight of the catalyst is 0.5 weight% or more and 1 weight% or less.

<Method for Preparing Catalyst>

The catalyst of the disclosure can be prepared by general support methods. For example, the catalyst of the disclosure can be prepared by: (1) the step of dissolving a palladium (Pd) compound (such as palladium (Pd) oxide or chloride) in an appropriate solvent (water, an aqueous acid solution, an aqueous alkali solution, an organic solvent, or the like) to obtain a palladium (Pd) compound solution; (2) the step of depositing the palladium (Pd) compound derived from the solution prepared in (1) above on a surface of a titanium oxide (TiO$_2$) support; and (3) the step of reducing the palladium (Pd) compound deposited on the surface of the titanium dioxide (TiO$_2$) support to metal palladium (Pd) with hydrogen, hydrazine, formaldehyde, and/or the like to obtain a catalyst in which the palladium (Pd) is supported on the titanium dioxide (TiO$_2$) support. The step (2) above can be performed, for example, by a method of spraying the solution prepared in (1) above onto the titanium dioxide (TiO$_2$) support and then drying the solution; a method of immersing the titanium dioxide (TiOz) support in the solution prepared in (1) above and allowing the palladium (Pd) compound to be adsorbed onto the titanium dioxide (TiOz) support, and then heating and evaporating the solvent; or the like. An example of the palladium (Pd) compound is palladium (II) acetylacetonate (Pd(acac)$_2$).

When the catalyst of the disclosure contains the sulfur trioxide (SO$_3$), for example, the sulfur trioxide (SO$_3$) may be mixed in the catalyst of the disclosure as a trace component, an impurity, or the like contained in the titanium dioxide (TiO$_2$) support during the preparation of the titanium dioxide (TiOz) support. Alternatively, after the preparation of the titanium dioxide (TiO$_2$) support, the sulfur trioxide (SO$_3$) may be treated to be contained in the titanium dioxide (TiOz) support. In a case in which there is a commercially available product of the titanium dioxide (TiOz) support that contains the sulfur trioxide (SO$_3$) as a trace component, an impurity, or the like, such a commercially available product may be used as the titanium dioxide (TiOz) support.

The catalyst of the disclosure is preferably reduced (e.g., hydrogenation reduction) prior to use in a reaction.

<CPD Hydrogenation Reaction>

A reaction to be promoted by the catalyst of the disclosure is a hydrogenation reaction (CPD hydrogenation reaction) of CPD to produce CPE, which takes place in a gas phase. Preferred conditions for the CPD hydrogenation reaction will be described in the "Method for Producing CPE" section below.

(Method for Producing CPE)

A method for producing CPE according to the disclosure includes the step (hydrogenation step) of conducting a hydrogenation reaction of CPD in a gas phase to form the CPE in the presence of the above-described catalyst.

The method of the disclosure may further include the following steps, as optional steps to be performed before the hydrogenation step:

· the step of reducing the catalyst (catalyst reduction step);
· the step of vaporizing a reaction raw material (CPD, multimerized CPD, or a mixture of CPD and coexisting components such as the solvent) (vaporization step); and/or
· the step of heating multimerized CPD to obtain monomerized CPD by thermal decomposition (thermal decomposition step).

The method of the disclosure may also further include the following step, as an optional step to be performed after the hydrogenation step:
· the step of cooling objective CPE to liquefy or solidify the objective CPE (cooling step).

<Hydrogenation Step>

The hydrogenation step is the step of conducting the hydrogenation reaction of the CPD in the gas phase to form the CPE in the presence of the above-described catalyst.

<<Reaction Substrate>>

The CPD, which is a substrate for the hydrogenation reaction, may be used in the hydrogenation step as a simple substance, or may be used in the hydrogenation step in the form of a mixture. As the mixture, there are mixtures with the CPE and mixtures with common organic solvents (e.g., toluene or hexane). The CPD may be a commercial product or a fraction (e.g., naphtha cracker Cs fraction) of a petroleum material. In particular, a fraction of a petroleum material may be used as a CPD-CPE mixture.

When the CPD is in the form of the CPD-CPE mixture, the molar ratio (CPE/CPD) of CPE relative to CPD in the mixture is not particularly limited, but is preferably 0.0485 or more, more preferably 0.0970 or more, and even more preferably 0.194 or more, preferably 29.1 or less, more preferably 19.4 or less, and even more preferably 9.70 or less. When the molar ratio (CPE/CPD) of the amount of the CPE added to the CPD in the hydrogenation step is equal to or above these lower limits, multimerization of the CPD can be suppressed and the purity and yield rate of the obtained CPE can be further increased. On the other hand, when the molar ratio (CPE/CPD) of the amount of the CPE added to the CPD in the hydrogenation step is equal to or below these upper limits, the productivity of the CPE can be sufficiently ensured.

The molar ratio (hydrogen/CPD) of the amount of hydrogen added to the CPD in the hydrogenation step is not particularly limited, but is preferably 1 or more, preferably 20 or less, and more preferably 10 or less.

A mixture of multimerized CPD and the CPE may also be used as a reaction raw material for the method of the disclosure. The multimerized CPD in such a reaction raw material is decomposed to monomerized CPD by a thermal decomposition step described below and become a substrate for the hydrogenation reaction. As such a reaction raw material, for example, a solution of the multimerized CPD (e.g., dicyclopentadiene) diluted with the CPE to 70 weight% to 25 weight% can be used.

In the reaction raw material for the method of the disclosure, the content of a sulfur component is preferably 5 ppm or less, more preferably 0.1 ppm or less, and no sulfur component is most preferable. The presence of the sulfur component in the reaction raw material is disadvantageous in terms of shortening the life of the catalyst.

<<Temperature of Hydrogenation Step>>

The hydrogenation step is performed in the gas phase. The hydrogenation step is performed "in the gas phase" means that the hydrogenation step is performed while substances in a hydrogenation reaction system other than the catalyst are in a gaseous state. The substances in the hydrogenation reaction system other than the catalyst include substances before the hydrogenation reaction such as hydrogenation substrate (CPD), coexisting components (when the hydrogenation substrate is in the form of a mixture), and hydrogen, and substances after the hydrogenation reaction such as hydrides (CPE), coexisting components (when the hydrides are in the form of a mixture), and excess hydrogen. By conducting the hydrogenation step in the gas phase, productivity can be improved with fewer number of devices.

The temperature in the hydrogenation step is not limited as long as the purpose of the disclosure is achieved. However, from the viewpoint that a low reaction temperature leads to the precipitation of partially liquefied polymerized CPD and clogging in the reaction tube, the temperature in the hydrogenation step is preferably 130°C or more, and more preferably 150°C or more. The temperature in the hydrogenation step is also preferably 350°C or less, and more preferably 250°C or less from the viewpoint of suppressing degradation in the catalyst and a reaction device and suppressing a hydrogenation overreaction (decrease in selectivity).

«Shape etc. of Reactor»

A reactor to be used in the hydrogenation step can be either a continuous type or a batch type, but the continuous type is preferable from the viewpoint of improving productivity. The reactor to be used in the hydrogenation step is not limited by its shape, but is preferably a multi-tube fixed bed distribution reactor. When the multi-tube fixed bed distribution reactor is used, the inner diameter of reaction tubes is not particularly limited, but is preferably 6 mm or more, more preferably 10 mm or more, preferably 100 mm or less, and more preferably 70 mm or less. The length of the reaction tubes is not particularly limited, but is preferably 0.1 m or more, more preferably 0.3 m or more, preferably 10 m or less, and more preferably 7 m or less. The reactor used in the hydrogenation step is preferably a reaction tube of 1 inch or more for 2 to 4 mm of the catalyst.

<<Reaction Pressure>>

The pressure (total pressure) during the reaction is usually 0 MPa to 0.1 MPa, preferably 0 MPa to 0.05 MPa. High reaction pressure may cause selectivity loss due to gas penetration into the catalyst.

<<Introduction of Hydrogen>>

The introduction of a hydrogen gas into the reaction tubes is preferably down flow. The amount of the hydrogen gas fed into the reaction tubes is usually preferably 1 equivalent to 2.0 equivalents of the source CPD, and more preferably 1 equivalent to 1.2 equivalents. It is preferable that the amount of hydrogen is not too much, since a large amount of hydrogen causes entrainment and a large cooler for product recovery.

<<Other Components>>

In the hydrogenation step, components (other components) other than the catalyst, the CPD, and components mixed with the CPD can be present. Specifically, the other components include, for example, inactive gases such as nitrogen and argon.
One type of the other component may be used alone, or two or more types may be used in combination in any ratio.

«Installation Style of Catalyst»

In the hydrogenation step, the catalyst is usually installed in a reaction vessel. An installation style of the catalyst is not particularly limited as long as the purpose of the disclosure is achieved, but for example, may be installed as a fixed bed catalyst in the reaction vessel. The amount of the catalyst installed in the reactor vessel per volume of the reaction vessel is not particularly limited as long as the purpose of the disclosure is achieved, but may be, for example, 500 g/L or more, preferably 750 g/L or more, and may be, for example, 150 g/L or less, and preferably 125 g/L or less.

<Catalyst Reduction Step>

In order to activate the catalyst in a hydrogenation reaction step, the method of the disclosure preferably further includes the step (catalyst reduction step) of reducing the catalyst before the hydrogenation reaction step. The catalyst reduction step can be performed, for example, by heating the catalyst in a hydrogen atmosphere. The heating temperature may be, for example, 150°C or more, preferably 300°C or more, and may be, for example, 450°C or less, and preferably 400°C or less.

<Vaporization Step>

When the reaction raw material (CPD, multimerized CPD, or a mixture of CPD with coexisting components such as the solvent) is liquid or solid at room temperature, the method of the disclosure preferably further includes the step (vaporization step) of vaporizing the reaction raw material before the hydrogenation reaction step, in order to conduct the hydrogenation step in a gas phase. The vaporization step may be performed, for example, by heating or depressurizing the reaction raw material, but is usually performed by heating the reaction raw material. The heating temperature may be any temperature as long as the reaction raw material vaporizes and the deterioration of the reaction device is suppressed, and may be, for example, 150°C or more, preferably 190°C or more, and may be, for example, 250°C or less, and preferably 210°C or less.

<Thermal Decomposition Step>

The substrate (CPD) for the hydrogenation reaction may form multimers (e.g., dimers) in the reaction raw material. For example, the CPD tend to exist in the form of dicyclopentadiene (DCPD), which is dimerized to dimers, at room temperature. Therefore, it is preferable that the method of the disclosure further includes, prior to the hydrogenation reaction step, the step (thermal decomposition step) of heating the multimerized CPD to obtain the CPD as monomers through thermal decomposition. The thermal decomposition step may be performed after the vaporization step, before the vaporization step, or integrally with the vaporization step. The heating temperature may be any temperature as long as the multimerized CPD is monomerized and the deterioration of the reaction device is suppressed, and may be, for example, 340°C or more, preferably 350°C or more, and may be, for example, 250°C or less, and preferably 360°C or less.

<Cooling Step>

When the target CPE is liquid or solid at room temperature, the method of the disclosure preferably further includes the step (cooling step) of cooling the CPE to liquefy or solidify the CPE, after the hydrogenation reaction step, in order to recover the CPE obtained in the hydrogenation step as liquid or solid. The cooling temperature may be any temperature as long as the CPE is liquefied or solidified, and may be, for example, 0°C or less, and preferably - 20°C or less. The cooling step may be performed by a method known in the art, for example, using a condenser.

<Other Steps>

The method of the disclosure may further include the following step, as another optional step to be performed before the hydrogenation step:
· the step of removing an impurity (for example, multimerized CPD that has not been monomerized, or a byproduct of the thermal decomposition step) from the CPD by distillation or the like (reaction raw material impurity removal step).
The method of the disclosure may further include the following step, as another optional process to be performed after the hydrogenation step:
• the step of removing an impurity (for example, CPD that has not been hydrogenated, or a byproduct of the hydrogenation step, such as perhydroxides,) from the target CPE by distillation or the like (product impurity removal step).

<Reaction Device>

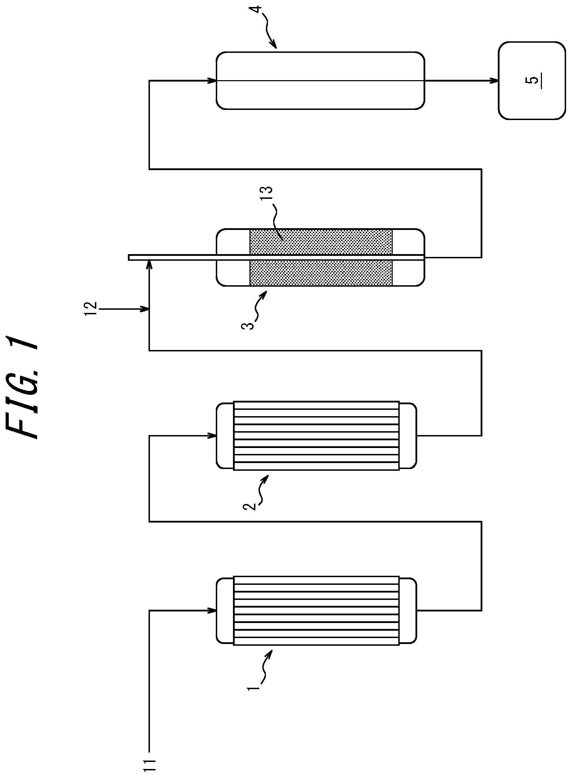

FIG. 1 illustrates an example of the reaction device used in the method of the disclosure when the method of the disclosure includes the vaporization step, the thermal decomposition step, the hydrogenation step, and cooling step.

<Suitable Conversion Rate and Selectivity Rate>

The conversion rate from the CPD to the CPE that can be achieved by the method of the disclosure is preferably 95% or more, more preferably 99% or more, and even more preferably 99.8% or more. The selectivity rate from the CPD to the CPE that can be achieved by the method of the disclosure is preferably 90% or more, more preferably 92% or more, and even more preferably 96% or more.

EXAMPLES

The disclosure will be specifically described below based on the examples, but the disclosure is not limited to these examples. In the following description, "%" and "part" representing amounts are on a mass basis, unless otherwise specified.
In Examples and Comparative Examples, a cyclopentadiene (CPD) conversion rate, a cyclopentene (CPE) selectivity rate, and a CPE yield rate were calculated by the following methods. The composition of a reaction solution was measured by gas chromatography.

<$SO_3$ Content>

In the Examples and Comparative Examples, the content of sulfur trioxide ($SO_3$) was measured by X-ray fluorescence analysis (XRF).

<CPD Conversion Rate>

The CPD conversion rate in the hydrogenation step in the Examples and Comparative Examples was calculated by the following equation (1):

$$\text{CPD conversion rate (\%)} = 100-(F1/F2)\times100 \quad \cdots (1)$$

In the equation (1), F1 is the flow rate (g/hour) of CPD at an outlet of a reaction tube, and F2 is the flow rate (g/hour) of the CPD introduced into the reaction tube.

<CPE Selectivity Rate>

The CPE selectivity rate in the hydrogenation step in the Examples and Comparative Examples was calculated by the following equation (2):

$$\text{CPE selectivity rate (\%)} = \{(F3-F4)/F5\}\times100 \quad \cdots (2)$$

In the equation (2), F3 is the flow rate (g/hour) of CPE at the outlet of the reaction tube, F4 is the flow rate (g/hour) of the CPE introduced into the reaction tube (F4=0 when the gas-phase contact hydrogenation of the CPD is performed in the absence of the CPE), and F5 is the flow rate (g/hour) of the converted CPD. The higher the CPE selectivity rate, the higher the purity of the CPE obtained in the hydrogenation step.

<CPE Yield Rate>

The CPE yield rate in the hydrogenation step in the Examples and Comparative Examples was calculated by the following equation (3) using the CPD conversion rate and the CPE selectivity rate described above:

$$\text{CPE yield rate (\%)} = (\text{CPD conversion rate (\%)} \times \text{CPE selectivity rate (\%)})/100 \quad \cdots (3)$$

The higher this CPE yield rate, the higher the yield rate of the CPE obtained in the hydrogenation step.

[Example 1]

(Production of Pd-bearing Support for Hydrogenation Catalyst)

A TiOz support with an anatase ratio of 100% was impregnated in a Pd-containing organic solvent, and then the impregnated support was dried to evaporate the solvent and sintered, to obtain a Pd-bearing support for a hydrogenation catalyst. The used TiOz support contained sulfur trioxide ($SO_3$) as a trace component. The obtained Pd-bearing support for the hydrogenation catalyst contained $SO_3$ at a ratio of 1.2% relative to the total weight of TiOz and the $SO_3$. Table 1 indicates the physical properties of the obtained Pd-bearing supports for the hydrogenation catalyst.

[Table 1]

| | | |
|---|---|---|
| Composition | TiO$_2$ (Content ratio % relative to total catalyst) | 98.4 |
| | Pd (Content ratio % relative to total catalyst) | 0.5 |
| | SO$_3$ (Content ratio % relative to total weight of TiO$_2$ and SO$_3$) | 1.2 |
| Physical properties of TiO$_2$ | Basic ($\mu$mol/g) @ 100 to 450°C | 0.109 |
| | Acidic ($\mu$mol/g) @ 100 to 450°C | 0.213 |
| Physical properties of Pd | Metal fineness of grind (%) | 1.07 |
| | Metal specific surface area (m$^2$/g·TiO$_2$ support) | 0.024 |
| | Metal specific surface area (m$^2$/g·Pd metal) | 4.77 |
| Physical properties of catalyst (Pd-bearing support) | Bet specific surface area (m$^2$/g) | 38.6 |
| | Total pore volume (cm$^3$/g) | 0.211 |
| | Average pore diameter (nm) | 21.9 |
| | Unit adsorption amount (cm$^2$/g) | 0.011 |
| | Particle diameter (nm) | 104.7 |

In the table, the unit adsorption amount of the catalyst was measured using the CO-pulse adsorption method.

(Production of CPE)

<Catalyst Reduction Step>

The Pd-bearing support for the hydrogenation catalyst was packed in a 1-inch tube, and subjected to reduction treatment under an $H_2$ atmosphere at a high temperature of 150°C to 450°C to convert the Pd-bearing support into an active hydrogenation catalyst.

<Vaporization Step • Thermal Decomposition Step>

Liquid DCPD, as a raw material, diluted with CPE was gasified at 190°C. The gas was heated to 350°C to decompose the DCPD into CPD (a reaction substrate) to obtain a CPD/CPE mixed gas.

<Hydrogenation Step • Cooling Step>

The CPD/CPE mixed gas (a flow rate of 0.3 to 0.5 ml/min) was distributed together with hydrogen (a flow rate of 100 to 150 ml/min) in a 1-inch tube filled with the catalyst (10 g) at a hydrogen equivalent (1.0 to 2.0) and a reaction temperature (150 to 250°C). The distributed gas was cooled at - 10°C with a condenser to obtain a CPE solution.

<Evaluation of Catalytic Properties>

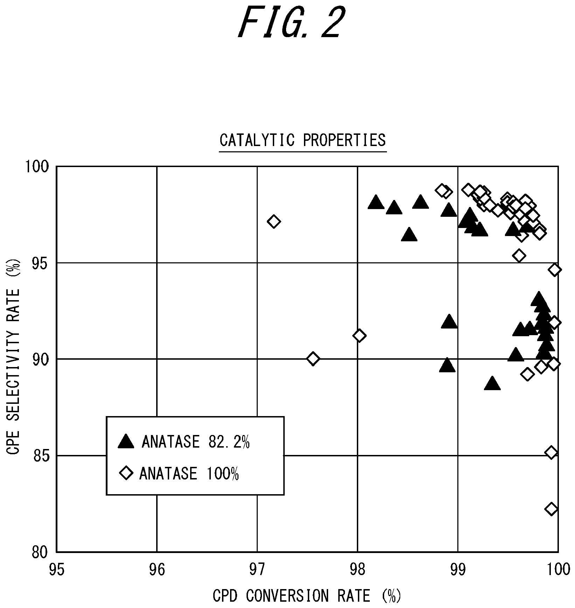

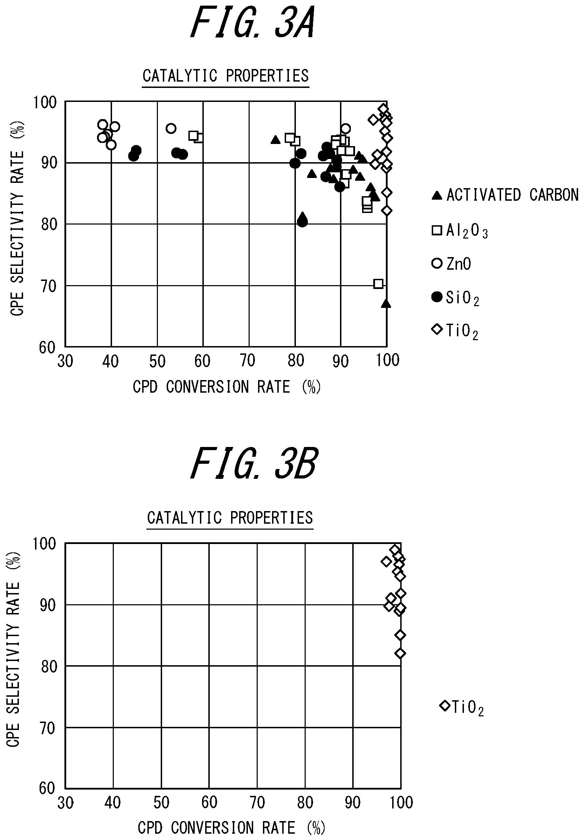

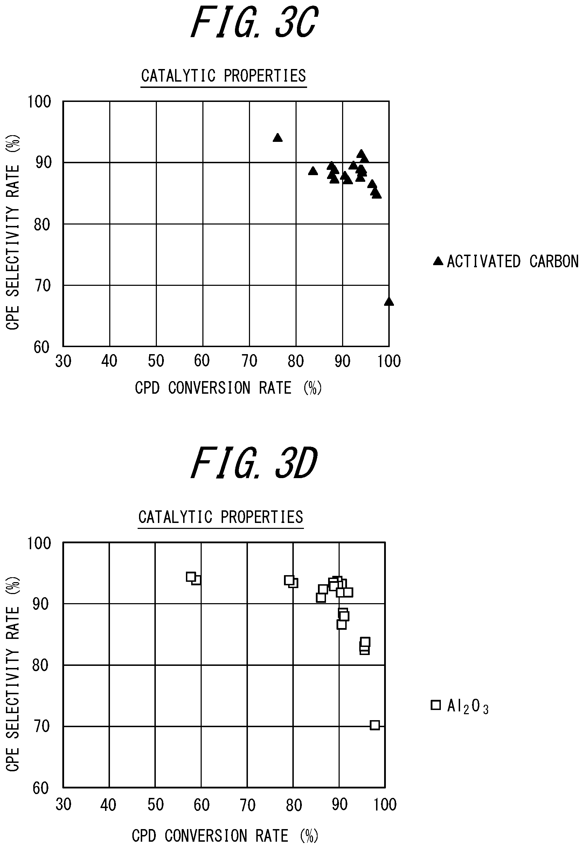

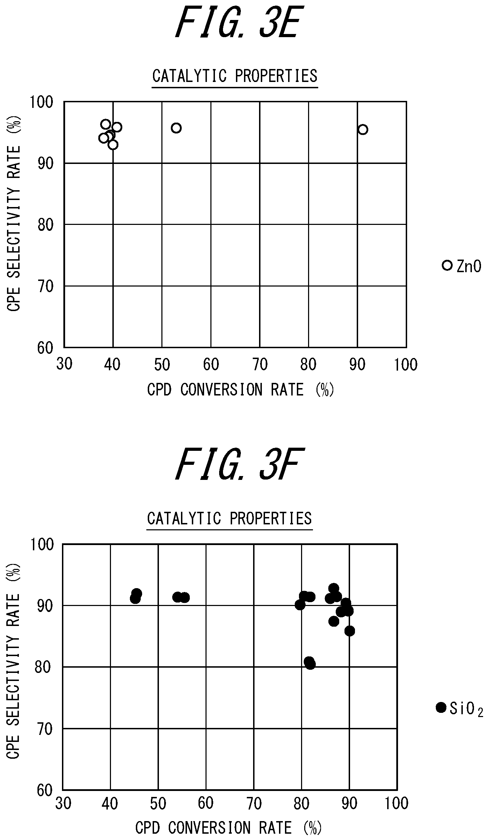

The CPD conversion rate, the CPE selectivity rate, and the CPE yield rate were evaluated. FIGS. 2 and 3 indicate the results, and Table 2 indicates representative values of the results.

[Example 2]

The test was conducted in the same manner as in Example 1, except that TiOz with an anatase ratio of 82.2% was used as a support, instead of the TiOz with an anatase ratio of 100% used in Example 1. The used TiOz support contained sulfur trioxide ($SO_3$) as a trace component. The obtained Pd-bearing support for a hydrogenation catalyst contained $SO_3$ at a ratio of 0.9% relative to the total weight of the TiOz and the $SO_3$. FIG. 2 and Table 2 indicate the results.

[Example 3]

The test was conducted in the same manner as in Example 1, except that TiOz with an anatase ratio of 100% without containing $SO_3$ was used as a support, instead of the TiOz support used in Example 1. The obtained Pd-bearing support for a hydrogenation catalyst also did not contain $SO_3$. Table 2 indicates the results.

[Comparative Examples 1 to 4]

The tests were conducted in the same manner as in Example 1, except that activated carbon (Comparative Example 1), aluminum oxide ($Al_2O_3$) (Comparative Example 2), zinc oxide (ZnO) (Comparative Example 3), or silicon dioxide (SiOz) (Comparative Example 4) was used, instead of titanium dioxide (TiOz), as a support in the production of hydrogenation catalysts. FIG. 3 and Table 2 indicate the results.

[Table 2]

| Example/ Comparative Example No. | Example 1 | Example 2 | Example 3 | Comparative Example 1 | Comparative Example 2 | Comparative Example 3 | Comparative Example 4 |
|---|---|---|---|---|---|---|---|
| Support | TiO$_2$ (anatase ratio of 100%) | TiO$_2$ (anatase ratio of 82.2%) | TiO$_2$ (anatase ratio of 100%) | Activated carbon | Al$_2$O$_3$ | ZnO | SiO$_2$ |
| Content ratio of SO$_3$ | 1.20% | 0.90% | - | - | - | - | - |
| CPD conversion rate | 99.50% | 99.30% | 99.70% | 97.40% | 98.20% | 91.00% | 90.00% |
| CPE selectivity rate | 98.20% | 88.80% | 87.60% | 84.60% | 70.20% | 95.60% | 86.20% |
| CPE yield rate | 97.70% | 88.20% | 87.40% | 82.40% | 69.00% | 87.00% | 77.60% |

The results in Table 2 indicate that the Pd-supported catalyst that uses the titanium dioxide (TiOz) as the support has superior catalytic properties such as the CPD conversion rate, the CPE selectivity rate, and the CPE yield rate. It is also indicated that the higher the anatase ratio of the titanium dioxide ($TiO_2$), the better the catalytic properties. It is also indicated that the presence of the $SO_3$ in the catalysts gives even better catalytic properties.

INDUSTRIAL APPLICABILITY

The disclosure enables a hydrogenation reaction of CPD to CPE in a gas phase that exhibits both a high conversion rate and high selectivity.

REFERENCE SIGNS LIST

1     vaporizer
2     decomposition reactor
3     hydrogenation reactor
4     cooler
5     reaction liquid tank
11    raw material inlet
12    hydrogen inlet
13    catalyst charging and reaction point

**Claims**

1. A catalyst used in a hydrogenation reaction of cyclopentadiene in a gas phase to form cyclopentene, the catalyst comprising:

   palladium (Pd); and
   a titanium dioxide (TiOz) support,
   wherein the titanium dioxide (TiOz) support contains anatase-type titanium dioxide (TiOz).

2. The catalyst according to claim 1, wherein

   (i) a content of titanium dioxide (TiOz) relative to a total weight of the catalyst is 85 weight% or more and 99.9 weight% or less, and
   (ii) a content of the palladium (Pd) relative to a weight of the titanium dioxide (TiOz) support is 0.1 weight% or more and 2 weight% or less.

3. The catalyst according to claim 1 or 2, wherein

   (i') a content of titanium dioxide ($TiO_2$) relative to a total weight of the catalyst is 95 weight% or more and 99.9 weight% or less, and
   (ii') a content of the palladium (Pd) relative to a weight of the titanium dioxide (TiOz) support is 0.5 weight% or more and 1 weight% or less.

4. The catalyst according to any one of claims 1 to 3, wherein titanium dioxide ($TiO_2$) has an anatase ratio of 80% or more.

5. The catalyst according to any one of claims 1 to 4, wherein titanium dioxide ($TiO_2$) has an anatase ratio of 95% or more.

6. The catalyst according to any one of claims 1 to 5, further comprising sulfur trioxide ($SO_3$).

7. The catalyst according to claim 6, wherein a content of the sulfur trioxide ($SO_3$) relative to a total weight of titanium dioxide (TiOz) and the sulfur trioxide ($SO_3$) in the catalyst is 0.1 weight% or more and 5.0 weight% or less.

8. A method for producing cyclopentene comprising conducting a hydrogenation reaction of cyclopentadiene in a gas phase to form the cyclopentene in the presence of the catalyst according to any one of claims 1 to 7.

# FIG. 1

EP 4 316 649 A1

# FIG. 2

CATALYTIC PROPERTIES

## FIG. 3A

CATALYTIC PROPERTIES

▲ ACTIVATED CARBON

□ $Al_2O_3$

○ ZnO

● $SiO_2$

◇ $TiO_2$

## FIG. 3B

CATALYTIC PROPERTIES

◇ $TiO_2$

## FIG. 3C

CATALYTIC PROPERTIES

▲ ACTIVATED CARBON

## FIG. 3D

CATALYTIC PROPERTIES

□ $Al_2O_3$

# FIG. 3E

CATALYTIC PROPERTIES

○ ZnO

# FIG. 3F

CATALYTIC PROPERTIES

● SiO$_2$

**EP 4 316 649 A1**

## INTERNATIONAL SEARCH REPORT

International application No.

**PCT/JP2022/013503**

### A. CLASSIFICATION OF SUBJECT MATTER

***B01J 23/44***(2006.01)i; ***B01J 35/10***(2006.01)i; ***C07B 61/00***(2006.01)i; ***C07C 5/05***(2006.01)i; ***C07C 13/12***(2006.01)i
FI: B01J23/44 Z; C07C13/12; C07C5/05; B01J35/10 301A; C07B61/00 300

According to International Patent Classification (IPC) or to both national classification and IPC

### B. FIELDS SEARCHED

Minimum documentation searched (classification system followed by classification symbols)

B01J21/00-38/74; C07B31/00-63/04; C07C1/00-409/44

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); Scopus; JSTChina (JDreamIII)

### C. DOCUMENTS CONSIDERED TO BE RELEVANT

| Category* | Citation of document, with indication, where appropriate, of the relevant passages | Relevant to claim No. |
|---|---|---|
| Y | JP 2003-137821 A (MARUZEN PETROCHEM CO LTD) 14 May 2003 (2003-05-14)<br>paragraphs [0015], [0040]-[0041] | 1-8 |
| Y | JP 2019-518602 A (VENATOR GERMANY GMBH) 04 July 2019 (2019-07-04)<br>paragraphs [0002]-[0003], [0010], [0031] | 1-8 |
| Y | JP 2013-510707 A (MILLENNIUM INORGANIC CHEMICALS, INC) 28 March 2013<br>(2013-03-28)<br>paragraph [0020] | 6-7 |
| A | JP 2000-053597 A (NIPPON ZEON CO LTD) 22 February 2000 (2000-02-22)<br>entire text | 1-8 |
| A | JP 2001-181217 A (NIPPON ZEON CO LTD) 03 July 2001 (2001-07-03)<br>entire text | 1-8 |
| A | JP 2001-010987 A (NIPPON ZEON CO LTD) 16 January 2001 (2001-01-16)<br>entire text | 1-8 |

☐ Further documents are listed in the continuation of Box C. ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **06 May 2022** | **17 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2022/013503**

| Patent document cited in search report | | | Publication date (day/month/year) | Patent family member(s) | | | Publication date (day/month/year) |
|---|---|---|---|---|---|---|---|
| JP | 2003-137821 | A | 14 May 2003 | (Family: none) | | | |
| JP | 2019-518602 | A | 04 July 2019 | US 2017/0348671 A1 paragraphs [0002]-[0003], [0012], [0058] US 2020/0306727 A1 WO 2017/211710 A1 EP 3464184 A1 TW 201808813 A CA 3025085 A AU 2017277063 A CN 109311695 A KR 10-2019-0017898 A BR 112018073994 A EA 201892791 A | | | |
| JP | 2013-510707 | A | 28 March 2013 | US 2011/0118109 A1 paragraph [0022] US 2012/0283092 A1 WO 2011/059606 A2 EP 2499096 A1 CA 2778778 A CN 102596813 A AU 2010318634 A TW 201132592 A MX 2012005244 A KR 10-2012-0101386 A UA 107946 C SA 3400 B BR 112012011278 A | | | |
| JP | 2000-053597 | A | 22 February 2000 | (Family: none) | | | |
| JP | 2001-181217 | A | 03 July 2001 | (Family: none) | | | |
| JP | 2001-010987 | A | 16 January 2001 | (Family: none) | | | |

Form PCT/ISA/210 (patent family annex) (January 2015)

20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S5476549 U
- JP 2001261592 A

- JP 2004175685 A